# EUROPÄISCHE PATENTSCHRIFT

(11) **EP 0 736 095 B1**
(45) Veröffentlichungstag und Bekanntmachung des Hinweises auf die Patenterteilung: **05.06.2002**
(21) Anmeldenummer: 95905105.3
(22) Anmeldetag: 22.12.1994
(51) Int. Cl.: C12N 15/19, C07K 14/52, A61K 38/19, G01N 33/53

(54) **HUMANES ZIRKULIERENDES CYTOKIN CC-1**
HUMAN CIRCULATING CYTOKINE CC-1
CYTOKINE HUMAINE CIRCULANTE CC-1

(30) Priorität: 24.12.1993 DE 4344397; 03.08.1994 DE 4427395
(43) Veröffentlichungstag der Anmeldung: 09.10.1996
(73) Patentinhaber: Forssmann, Wolf-Georg, 30175 Hannover (DE)
(72) Erfinder: FORSSMANN, Wolf-Georg, D-30175 Hannover (DE); SCHULZ-KNAPPE, Peter, D-30625 Hannover (DE); MEYER, Markus, D-30625 Hannover (DE); MÄGERT, Hans-Jürgen, D-30163 Hannover (DE)
(74) Vertreter: Meyers, Hans-Wilhelm, Dr.Dipl.-Chem.
(86) Internationale Anmeldenummer: EP9404282
(87) Internationale Veröffentlichungsnummer: WO9518228

(56) Entgegenhaltungen:
- WO-A-93/13206
- THE FASEB JOURNAL, Bd.3, Dezember 1989, BETHESDA,MD,USA Seiten 2565 - 2573 WOLPE ET AL 'MACROPHAGE INFLAMMATORY PROTEINS 1 AND 2:MEMBERS OF A NOVEL SUPERFAMILY OF CYTOKINES'

### SEQUENZPROTOKOLL

(1) ALLGEMEINE ANGABEN:
   (i) ANMELDER:
      (A) NAME: Prof. Dr. Wolf-Georg Forssmann
      (B) STRASSE: Bluecherstrasse 5
      (C) ORT: Hannover
      (E) LAND: Deutschland
      (F) POSTLEITZAHL: 30175
   (ii) BEZEICHNUNG DER ERFINDUNG: Humanes zirkulierendes Cytokin CC-1
   (iii) ANZAHL DER SEQUENZEN: 9
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patentin Release #1.0, Version #1.30 (EPA)
(2) ANGABEN ZU SEQ ID NO: 1:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR-Primer"
   (iii) HYPOTHETISCH: NEIN
   (iv) ANTISENSE: NEIN
   (xi) SEQUENZBESCHREIBUNG: SEQ ID NO: 1:
   GCCCGGAATT CTAGACARCG NATHATGGAY TA 32
(2) ANGABEN ZU SEQ ID NO: 2:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR-Primer"
   (iii) HYPOTHETISCH: NEIN
   (iv) ANTISENSE: NEIN
   (xi) SEQUENZBESCHREIBUNG: SEQ ID NO: 2:
   CCCGAATTCT AGAARTAYCC NATHCCNCGN CA 32
(2) ANGABEN ZU SEQ ID NO: 3:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR-Primer"
   (iii) HYPOTHETISCH: NEIN
   (iv) ANTISENSE: NEIN
   (xi) SEQUENZBESCHREIBUNG: SEQ ID NO: 3:
   GCCCGGAATT CTAGACARAG RATHATGGAY TA 32
(2) ANGABEN ZU SEQ ID NO: 4:
   (i) SEQUENZKENNZEICHEN:
      (A) LÄNGE: 12 Aminosäuren
      (B) ART: Aminosäure
      (C) STRANGFORM: Einzelstrang
      (D) TOPOLOGIE: linear
   (ii) ART DES MOLEKÜLS: Peptid
   (iii) HYPOTHETISCH: NEIN
   (iv) ANTISENSE: NEIN
   (xi) SEQUENZBESCHREIBUNG: SEQ ID NO: 4:
   Lys Tyr Pro Ile Pro Arg Glu Arg Ile Met Asp Tyr
   1 5 10
(2) ANGABEN ZU SEQ ID NO: 5:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR-Primer"
   (iii) HYPOTHETISCH: NEIN
   (iv) ANTISENSE: NEIN
   (xi) SEQUENZBESCHREIBUNG: SEQ ID NO: 5:
   CCCGAATTCT AGAARTAYCC NATHCCNAGR CA 32
(2) ANGABEN ZU SEQ ID NO: 6:
   (i) SEQUENZKENNZEICHEN:
      (A) LÄNGE: 74 Aminosäuren
      (B) ART: Aminosäure
      (C) STRANGFORM: Einzelstrang
      (D) TOPOLOGIE: nicht bekannt
   (ii) ART DES MOLEKÜLS: Peptid
   (iii) HYPOTHETISCH: NEIN
   (iv) ANTISENSE: NEIN
   (xi) SEQUENZBESCHREIBUNG: SEQ ID NO: 6:
   Thr Lys Thr Glu Ser Ser Ser Arg Gly Pro Tyr His Pro Ser Glu Cys
   1               5                   10                  15
   Cys Phe Thr Tyr Thr Thr Tyr Lys Ile Pro Arg Gln Arg Ile Met Asp
               20                  25                  30
   Tyr Tyr Glu Thr Asn Ser Gln Cys Ser Lys Pro Gly Ile Val Phe Ile
           35                  40                  45
   Thr Lys Arg Gly His Ser Val Cys Thr Asn Pro Ser Asp Lys Trp Val
       50                  55                  60
   Gln Asp Tyr Ile Lys Asp Met Lys Glu Asn
   65                  70
(2) ANGABEN ZU SEQ ID NO: 7:
   (i) SEQUENZKENNZEICHEN:
      (A) LÄNGE: 123 Basenpaare
      (B) ART: Nucleotid
      (C) STRANGFORM: Einzelstrang
      (D) TOPOLOGIE: linear
   (ii) ART DES MOLEKÜLS: cDNA
   (iii) HYPOTHETISCH: NEIN
   (iv) ANTISENSE: NEIN
   (xi) SEQUENZBESCHREIBUNG: SEQ ID NO: 7:
   TATGAGACCA GCAGCCAGTG CTCCAAGCCC GGAATTGTCT TCATCACCAA AAGGGGCCAT    60
   TCCGTCTGTA CCAACCCCAG TGACAAGTGG GTCCAGGACT ATATCAAGGA CATGAAGGAG   120
   AAC                                                                 123
(2) ANGABEN ZU SEQ ID NO: 8:
   (i) SEQUENZKENNZEICHEN:
      (A) LÄNGE: 47 Aminosäuren
      (B) ART: Aminosäure
      (C) STRANGFORM: Einzelstrang
      (D) TOPOLOGIE: linear
   (ii) ART DES MOLEKÜLS: Peptid
   (iii) HYPOTHETISCH: NEIN
   (iv) ANTISENSE: NEIN
   (xi) SEQUENZBESCHREIBUNG: SEQ ID NO: 8:
   Gln Arg Ile Met Asp Tyr Tyr Glu Thr Asn Ser Gln Cys Ser Lys Pro
   1               5                   10                  15
   Gly Ile Val Phe Ile Thr Lys Arg Gly His Ser Val Cys Thr Asn Pro
               20                  25                  30
   Ser Asp Lys Trp Val Gln Asp Tyr Ile Lys Asp Met Lys Glu Asn
           35                  40                  45
(2) ANGABEN ZU SEQ ID NO: 9:
   (i) SEQUENZKENNZEICHEN:
      (A) LÄNGE: 330 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 9:
   GAATTCTAGA CAGCGGATCA TGGATTACTA TGAGACCAGC AGCCAGTGCT CCAAGCCCGG    60
   AATTGTCTTC ATCACCAAAA GGGGCCATTC CGTCTGTACC AACCCCAGTG ACAAGTGGGT   120
   CCAGGACTAT ATCAAGGACA TGAAGGAGAA CTGAGTGACC CAGAAGGGGT GGCGAAGGCA   180
   CAGCTCAGAG ACATAAAGAG AAGATGCCAA GGCCCCCTCC TCCACCCACC CCTAACTCTC   240
   AGCCCCAGTC ACCCTCTTGG AGCTTCCCTG CTTTGAATTA AAGACCACTC ATGCTCTTCA   300
   AAAAAAAAAA AAAAATGAGC TCTAGAATTC                                    330

## Beschreibung

Gegenstand der vorliegenden Erfindung ist ein Polypeptid aus der Klasse der Cytokine, Cytokin CC-1 sowie dessen biologisch aktive Fragmente und/oder Derivate, ein für das Cytokin CC-1 oder für seine biologisch aktiven Fragmente kodierendes Polynukleotid, insbesondere eine cDNA, ein Arzneimittel enthaltend das erfindungsgemäße Peptid, ein Diagnostikmittel, Verwendung von Cytokin CC-1 für zweite medizinische Indikationen sowie eine Nucleinsäuresonde hybridisierend für ein Polynukleotid kodierend für Cytokin CC-1 oder eines seiner Fragmente.

Es hat sich überraschenderweise gezeigt, daß sich aus humanem Hämofiltrat ein Cytokin CC-1 isolieren läßt. Das Cytokin hat die in SEQ ID No. 6 angegebene Aminosäuresequenz.

Auch Fragmente des Cytokins CC-1 weisen biologische Aktivität auf. Die Fragmente sind erhältlich durch dem Fachmann bekannte Methoden, beispielsweise durch Abdauung mit Peptidasen, insbesondere Endogroteasen. Auch Fragmentierung des erfindungsgemäßen Peptids mittels peptidbindungspaltender chemischer Reagenzien, insbesondere Cyanogenbromid liefert auch biologisch aktive Fragmente.

Das erfindungsgemäße Peptid ist erhältlich durch ein Isolationsverfahren ausgehend von humanem Hämofiltrat.

Das humane Hämofiltrat wird gegebenenfalls mit Wasser verdünnt und angesäuert. Der pH-Wert beträgt vorzugsweise 1,5 bis 3,5, insbesondere 2,5 bis 3,0. Danach wird das Hämofiltrat mit einem Kationenaustauscher behandelt, beispielsweise einem mit Sulfonsäuregruppen modifizierten Trägermaterial (Fractogel medium SO₃⁻ der Firma Merck). Die an den Kationenaustauscher gebundenen Peptide werden mit relativ hoch konzentrierter Salzlösung in einem sauren pH-Bereich, der dem obigen entspricht, eluiert. Die Ionenstärke der Elutionslösung entspricht dabei ungefähr einer 0,7 bis 1,3 molaren Natriumchloridlösung.

Das aufgefangene Eluat wird mit einem peptidfällenden Reagenz, beispielsweise Ammoniumsulfat versetzt. Die Ausfällung der Peptide erfolgt vorzugsweise bei niedrigeren Temperaturen, insbesondere im Bereich von 4 bis 10°C. Das so gewonnene Präzipitat wird von der überstehenden Lösung befreit, in Wasser aufgenommen und danach mit einem niederen peptidfällenden Alkohol, wie Isopropanol versetzt. Darin schließt sich eine weitere Kationenaustauscher-Chromatographie an. Diese Chromatographie ist vorzugsweise eine Gradientenelutions-Chromatographie mit einem Puffer geringer Ionenstärke bis zu einem Puffer mit höherer Ionenstärke entsprechend ungefähr einer Ionenstärke von 0,7 bis 1,3 M NaCl.

Die biologisch aktiven Fraktionen werden gepoolt und mittels präparativer Umkehrphasen-Chromatographie an mit C4 modifizierten Trägermaterialien weitergereinigt. Gegebenenfalls erfolgen weitere chromatographische Reinigungsschritte.

Die durch die chromatographische Reinigung erhaltene Substanz wurde der Strukturbestimmung zugeführt. Die Sequenzanalyse erfolgte über einen Edman-Abbau des Peptids sowie der Spalt produkte und wurden über einen ABI 473 A Sequenzer durchgeführt.

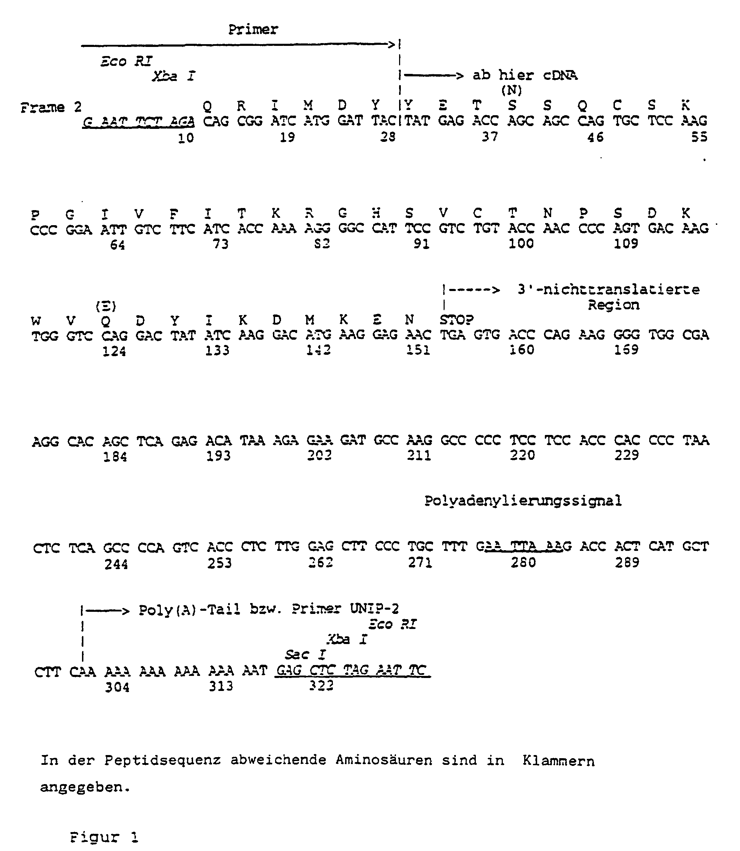

Aus der erfindungsgemäßen Peptidsequenz läßt sich ein Polynukleotid ableiten, kodierend für das Cytokin CC-1 (Fig. 1) mit dem C-terminalen Fragment gemäß SEQ ID No. 8 und der damit verbundenen Nukleinsäuresequenz SEQ ID No. 9.

Das Polynukleotid ist insbesondere eine cDNA, die sowohl als Ausgangspunkt einer gentechnischen Herstellung des Cytokins CC-1 dienen kann, wie auch als analytisches Werkzeug zum Nachweis des Auftretens von für das Protein kodierender DNA oder mRNA.

Dabei können entsprechende Derivate als Hybridisierungssonden eingesetzt werden. Beispielsweise weist die cDNA, die für ein Fragment des erfindungsgemäßen Peptids kodiert, eine Sequenz gemäß SEQ ID No. 7 auf.

Neben der gentechnischen Herstellung ist auch die aufbauende Totalsynthese an üblichen Festphasen im Sinne der Merrifield-Synthese möglich. Die Synthesenstrategie und der Aufbau des Peptids mit den entsprechend geschützten Aminosäuren sind dem Fachmann bekannt.

Das erfindungsgemäße Peptid kann als Arzneimittel Verwendung finden. Seine biologische Aktivität entspricht der eines Cytokins. Es ist daher geeignet, als Arzneimittel bei den in Anspruch 7 genannten Indikationen eingesetzt zu werden. Das erfindungsgemäße Peptid kann dabei in für Peptide üblicher Weise parenteral, intravenös oder intramuskulär oder intranasal, bukal verabreicht werden. Die Menge an zu verabreichenden Peptid beträgt zwischen 10 und 3.000 µg pro Darreichungseinheit.

Das erfindungsgemäße Diagnostikmittel enthält poly- oder monoklonale Antikörper gegen das erfindungsgemäße Peptid gegebenenfalls in fluoreszenz- oder radioaktivmarkierter Form um in an sich bekannten ELISA oder RIA Assays eingesetzt zu werden.

Die Erfindung wird anhand der folgenden Beispiele näher beschrieben.

### Beispiel 1

500 l humanes Hämofiltrat wurden mit Wasser auf 2.000 l verdünnt und mit konzentrierter HCl der pH auf 2,7 eingestellt. Nach Auftrag auf eine Amicon-Vantage Säule (Füllmaterial Merck Fractogel medium SO₃⁻) wurden die gebundenen Peptide mit 1 M NaCl pH 3,0 eluiert.

Das Eluat (7 l) wurde mit Ammoniumsulfat versetzt und über Nacht die Peptide bei 4°C ausgefällt. Das Peptidpräzipitat wurde über einen Büchner-Filter filtriert.

Das gewonnene Präzipitat wurde in 2 l Wasser gelöst und mit 4,5 Teilen Isopropanol versetzt. Die ausgefällten Peptide wurden erneut über einen Büchner-Filter abfiltriert.

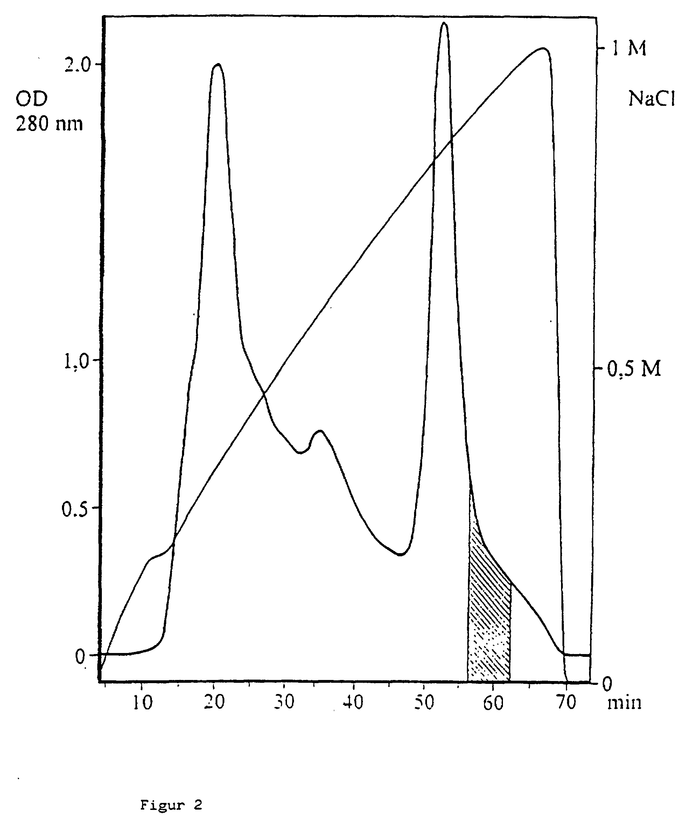

Das Präzipitat nach Isopropanol-Fällung wurde in 4 l Wasser gelöst und ein pH von 3,0 mit HCl eingestellt. Nach Auftrag auf einen Kationenaustauscher (Säule: Amicon Vantage) wurde die Säule eluiert und die Fraktionen gesammelt (Chromatogramm siehe Fig. 2).

### Chromatographie-Bedingungen

Puffer A: 10 mmol Natriumdihydrogenphosphat pH 3,0
Puffer B: Puffer A mit 1 M NaCl
Gradient: 0 - 100 % B in 60 min.
Fluß: 40 ml/min
Detektion: 280 nm
Chromatographieanlage: Biopilot (Pharmacia)
Fraktionen: à 2 min ab Start des Gradienten

Die Fraktionen 31 bis 34 wurden zur weiteren Behandlung vereinigt.

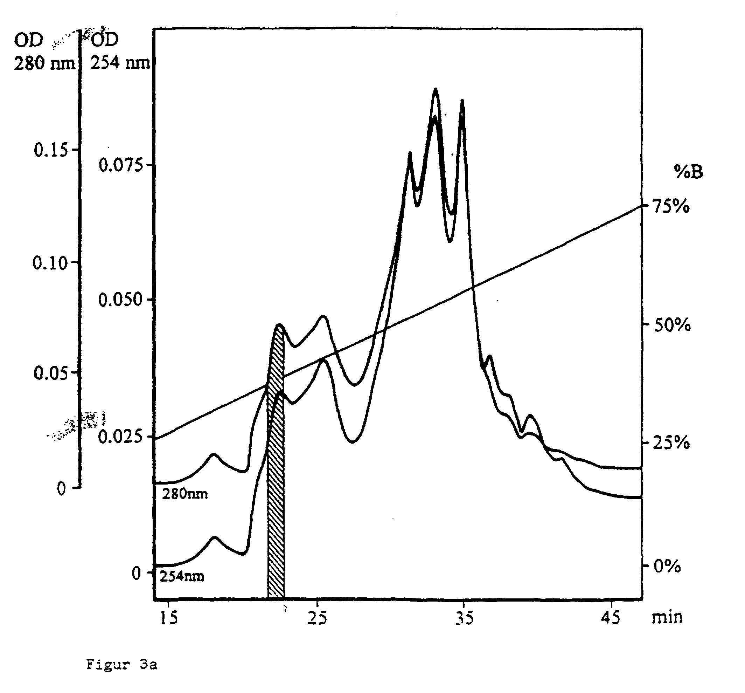

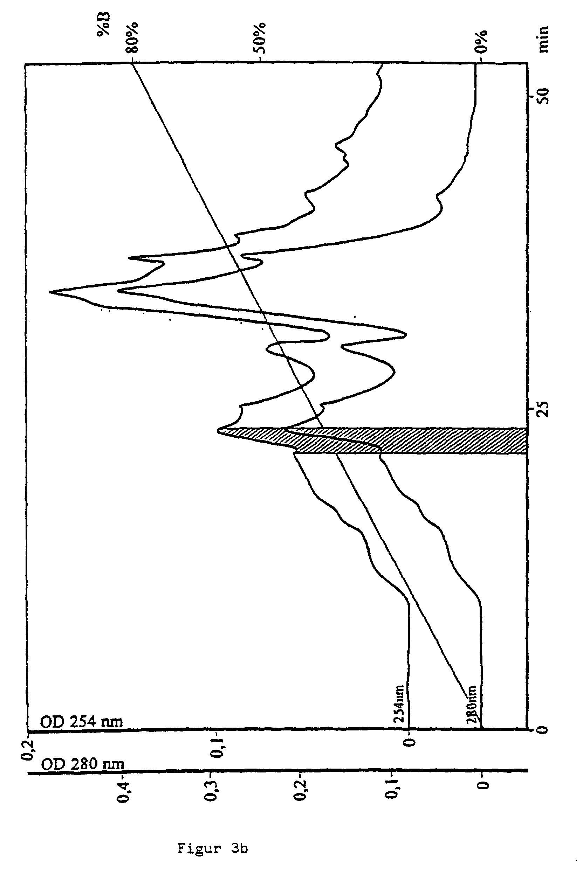

Die gepoolten Fraktionen 31 bis 34 wurden sukzessive in zwei Chromatographie-Läufen über eine präparative Reverse-Phase Säule aufgetrennt (Chromatogram siehe Fig. 3 a und b).

### Chromatographie-Bedingungen

Säule: 3 cm x 12,5 cm Stahlsäule
Füllmaterial: Parcosil RP-C4 25-45, 300 A
Puffer A: 0,01 N HCl
Puffer B: Puffer A mit 30 % Methanol und 50 % Isopropanol
Gradient: 0 - 100 % B in 60 min.
Fluß: 15 ml/min
Detektion: 280/254 nm
Chromatographieanlage: BioCAD (Perseptive)
Fraktionen: ä 1 min ab Start des Gradienten

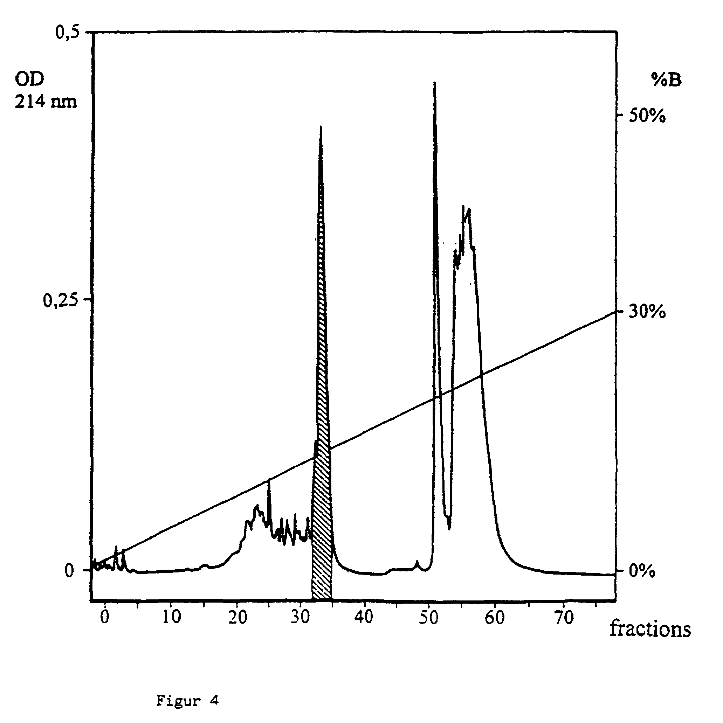

Die Fraktionen 22 und 23 aus dem ersten präparativen Lauf sowie die Fraktion 24 des zweiten Laufes wurden gepoolt und das Lösungsmittel über einen Rotationsverdampfer abgezogen. Danach wurden die Fraktionen über eine semipräparative RP-C4 Säule aufgetrennt (Chromatogram siehe Fig. 4).

### Chromatographie-Bedingungen

Säule: 1 cm x 12,5 cm Stahlsäule
Füllmaterial: Parcosil RP-C4 5 µ, 300 A
Puffer A: 0,1 % TFA
Puffer B: Puffer A mit 80 % Acetonitril
Gradient: 0 - 30 % B in 60 min.
Fluß: 2 ml/min
Detektion: 214 nm
Chromatographieanlage: Kontron 322
Fraktionen: à 1 min ab Start des Gradienten

Die Fraktion 33 und 34 enthält die zu über 95 % aufgereinigte Substanz, die im folgenden in ihrer Struktur aufgeklärt wurde:

### Beispiel 2

### Sequenz-Bestimmung

Edman-Abbau des Peptids sowie der Spaltprodukte erfolgte über einen ABI 473 A Sequenzer nach Auftragen auf eine Polybrene-Membran in Mengen zwischen 100 und 400 pmol unter Verwendung des Standard-Programmes.

### Bestimmung von Cysteinen

¹⁴C-Carboxymethylierung und nachfolgende Aufreinigung über analytische Vydac C18 RP-Säule (4,6 mm x 25 cm). Detektion der carboxymethylierten Fraktion im Radioaktivitätsmonitor.

Nachfolgend Lys-C-Spaltung von 50 % des carboxymethylierten Peaks mit der Endopeptidase Lys-C. Die Spaltung erfolgte bei 37°C über 3 Stunden in den vom Hersteller (Boehringer, Mannheim) angegebenen Puffern bei einem Verhältnis von Enzym zu Peptid von 1:25. Die Spaltprodukte wurden über RP-Chromatographie mit einer analytischen Vydac-C18-Säule getrennt. Sammeln der einzelnen Peaks und Sequenzierung zur Komplettbestimmung der Sequenz.

### Bestimmung des C-Terminus

Die Spaltung der restlichen 50 % des carboxymethylierten Peptids erfolgt mit Chymostrypsin in den vom Hersteller (Boehringer, Mannheim) angegebenen Puffern bei einem Verhältnis von Enzym zu Peptid von 1:25, die nachfolgende Aufreinigung über analytische Vydac C18 RP-Säule (4,6 mm x 25 cm). Die einzelnen Peaks werden gesammelt und zur Komplettbestimmung der Sequenz analysiert.

### Massenbestimmung

Massenbestimmung des Gesamtpeptids erfolgt mit Sciex API III sowie der Fragmente nach Lys-C- und Chymotrypsinspaltung.

sequenzierung und Massenbestimmung ergeben die oben angegebene Sequenz mit einer Masse von 8689 Dalton.

Ein Datenbankvergleich wurde durchgeführt an Swiss-Prot und EMBL-Peptid und Nukleinsäuredatenbank. Sequenzhomologie zu verschiedenen Mitgliedern der Superfamilie der Intercrine wurden festgestellt, dabei höchste Homologie zu Macrophage Inflammatory Protein MIP I alpha und MIP I beta.

### Beispiel 3

### Bestimmung der cDNA

### Klonierung und Charakterisierung eines partiellen humanen Cytokin CC-1 cDNA-Fragmentes

Aus humanen Nebennierengewebe wurde mit Hilfe eines automatischen Nukleinsäureextraktors (ABI,340) Gesamt-RNA präpariert.

Aus 5 µg dieser RNA wurde die mRNA unter Verwendung von MMLV-RTase (Gibco-BRL) und eines synthetischen Oligo(dT)-Primers (UNIP-2, CCTGAATTCTAGAGCTCA(T)₁₇) in cDNA-Erststrang umgeschrieben. Parallel dazu wurden ausgehend von der bekannten Peptid-Sequenz zwei "degenerierte" PCR-Primerpaare synthetisiert, welche sämtliche Codierungsmöglichkeiten für die entsprechenden Aminosäuresequenzen enthielten. Das erste Primerpaar (CC-1-2/1, CC-1-2/2) war dabei in Bezug auf die Aminosäuresequenz eher N-terminal lokalisiert, während das zweite Primerpaar (CC-1-2/3, CC-1-2/4) nach C-terminal verschoben positioniert war. Dies sollte eine Verstärkung in zwei Stufen (Vorverstärkung, Nachverstärkung) ermöglichen, um die Spezifität der Reaktion zu erhöhen. Folgende Reaktionen wurden durchgeführt:
1. In zwei verschiedenen Ansätzen wurden je 1/15 des cDNA-Ansatzes 40 PCR-Zyklen mit den Primerkombinationen CC-1-2/1 / UNIP-2 bzw. CC-1-2/2 / UNIP-2 unterzogen (Vorverstärkung, 2 Ansätze). Ein Zyklus bestand aus:

| | | |
|---|---|---|
| 95°C | 30 sec | Denaturierung |
| 48°C | 30 sec | Primer-Hybridisierung |
| 72°C | 3 min | Extension |

2. Je 1/30 der beiden Ansätze wurde danach mit den Primerkombinationen CC-1-2/3 / UNIP-2 bzw. CC-1-2/4 / UNIP-2 in 20 Zyklen nachamplifiziert (Nachverstärkung, 4 Ansätze):

| | | |
|---|---|---|
| 95°C | 30 sec | Denaturierung |
| 42°C | 30 sec | Primer-Hybridisierung |
| 72°C | 2 min | Extension |

Nachverstärkung mit CC-1-2/4 / UNIP-2 konnte ein homogenes PCR-Produkt erhalten werden. Der PCR-Ansatz wurde durch Centrikon C-100 (Amicon)- Zentrifugation von nicht umgesetzten Primern befreit, zusammen mit 50 ng pBluescript Eco-RI-restringiert (die PCR-Primer enthalten zur leichteren Klonierung Eco-RI-Schnittstellen) und anschließend ligiert. Die Ligationsprodukte wurden in E.coli XL-1 Blue propagiert, die Plasmid-DNA weißer Kolonien mit Qiagen-Säulen (Diagen) präpariert und mit Hilfe eines Fluoreszenzsequenzers sequenziert. Die klonierte cDNA kann nun als hochspezifische Hybridisiersonde zum Screening einer cDNA- oder Genbank eingesetzt werden. Ausgehend von der Sequenz können zudem spezifische Primer für eine direkte Amplifikation der restlichen cDNA aus Gesamt-DNA einer humanen cDNA-Bank abgeleitet werden.

| GAP-2-Aminosäuresequenz und abgeleitete PCR-Primer | | |
|---|---|---|
| Primer | | |
| CC-1-2/4 | SEQ ID No. 1, | +++! 48 Variationen |
| CC-1-2/1 | SEQ ID No. 2, | 768 Variationen |
| CC-1-2/3 | SEQ ID No. 3, | 24 Variationen |

| (kodierend für Fragment SEQ ID No. 4 GAP-2 AA-Seq.) | | |
|---|---|---|
| CC-1-2/2 | SEQ ID No. 5, | 384 Variationen |
| CC-1-2/3 | SEQ ID No. 6, | 24 Variationen |

## Patentansprüche

1. Cytokin CC-1 mit der Aminosäuresequenz gemäss SEQ ID No. 6 sowie dessen biologisch aktive amidierte, acetylierte, phosphorylierte und/oder glycosylierte Derivate.

2. Polynukleotid kodierend für Cytokin CC-1 nach Anspruch 1.

3. Polynukleotid nach Anspruch 2 **dadurch gekennzeichnet, dass** dieses ein cDNA-Fragment gemäss SEQ ID No. 7 ist.

4. Verfahren zur Herstellung eines Cytokins-CC-1 gemäss Anspruch 1 durch Extraktion von Hämofiltrat, Kationenaustauscher-Extraktion, gefolgt von Elution der adsorbierten Substanzen,
Ammoniumsulfat-Fällung der im Eluat vorhandenen Peptide und Proteine,
Aufnahme der Präzipitats in wässriger Lösung und erneute Fällung mit einem niederen Alkohol sowie Kationenaustauscher-Chromatographie, sowie Umkehrphasen-Chromatographie.

5. Arzneimittel enthaltend Cytokin CC-1 gemäss Anspruch 1 als wirksamen Bestandteil.

6. Diagnostikmittel enthaltend poly- oder monoklonale Antikörper gegen Cytokin CC-1 gemäss Anspruch 1 oder mit der für das Cytokin kodierenden Nukleinsäure oder mRNA.

7. Verwendung von Cytokin CC-1 gemäss Anspruch 1 zur Herstellung eines Arzneimittels zur Behandlung von Störungen der Migration von Zellen, Erkrankungen des Immunsystems, Tumoren sowie Disfunktion regulatorischer Wachstumsfunktionen.

## Claims

1. Cytokine CC-1 having the amino acid sequence according to SEQ ID NO.: 6 and its biologically active amidated, acetylated, phosphorylated and/or glycosylated derivatives.

2. A polynucleotide coding for cytokine CC-1 according to claim 1.

3. The polynucleotide according to claim 2, **characterized by** being a cDNA fragment according to SEQ ID NO.: 7.

4. A process for the preparation of cytokine CC-1 according to claim 1 by
extracting hemofiltrate by cation-exchange extraction followed by elution of the adsorbed substances;
ammonium sulfate precipitation of the peptides and proteins present in the eluate;
taking up the precipitate in aqueous solution and renewed precipitation with a lower alcohol, and cation-exchange chromatography, and reverse-phase chromatography.

5. A medicament containing cytokine CC-1 according to claim 1 as an active component.

6. A diagnostic agent containing poly- or monoclonal antibodies against cytokine CC-1 according to claim 1, or the nucleic acid or mRNA coding for said cytokine.

7. Use of cytokine CC-1 according to claim 1 for the preparation of a medicament for the treatment of disorders of the migration of cells, diseases of the immune system, tumors, and dysfunctions of regulatory growth functions.

## Revendications

1. Cytokine CC-1 comportant la séquence d'acides aminés d'après le SEQ ID N° 6 et ses dérivés amidifiés, acétylés, phosphorylés et/ou glycolysés biologiquement actifs.

2. Polynucléotide codant pour la cytokine CC-1 selon la revendication 1.

3. Polynucléotide selon la revendication 2, **caractérisé en ce que** celui-ci est un fragment d'ADNc d'après le SEQ ID N° 7.

4. Procédé de préparation d'une cytokine CC-1 selon la revendication 1 par extraction d'hémofiltrat, extraction par échange de cations, suivie d'une élution des substances adsorbées,
précipitation avec du sulfate d'ammonium des peptides et des protéines présents dans l'éluat,
recueil du précipité dans une solution aqueuse et précipitation renouvelée avec un alcool inférieur, ainsi que par chromatographie sur résine échangeuse de cations et chromatographie en phase inversée.

5. Médicament contenant de la cytokine CC-1 selon la revendication 1 en tant que composant actif.

6. Moyen de diagnostic comportant des anticorps poly- ou monoclonaux vis à vis de la cytokine CC-1 selon la revendication 1 ou contenant l'acide nucléique ou l'ARN-m codant pour la cytokine.

7. Utilisation de la cytokine CC-1 selon la revendication 1 pour préparer un médicament destiné au traitement des troubles de la migration cellulaire, des maladies du système immunitaire, des tumeurs ainsi que des troubles des fonctions de régulation de la croissance.
